# EUROPÄISCHE PATENTSCHRIFT

(11) **EP 0 852 595 B1**
(45) Veröffentlichungstag und Bekanntmachung des Hinweises auf die Patenterteilung: **02.06.1999**
(21) Anmeldenummer: 96933402.8
(22) Anmeldetag: 26.09.1996
(51) Int. Cl.: C08G 77/398, C08G 77/58

(54) **MESOSKOPISCHE ORGANOPOLYSILOXANPARTIKEL MIT CHEMISCH ANGEBUNDENEN METALLVERBINDUNGEN**
MESOSCOPIC ORGANOPOLYSILOXANE PARTICLES WITH CHEMICALLY BOUND METALLIC COMPOUNDS
PARTICULES D'ORGANOPOLYSILOXANE MESOSCOPIQUES PRESENTANT DES COMPOSES METALLIQUES CHIMIQUEMENT LIES

(30) Priorität: 28.09.1995 DE 19536182
(43) Veröffentlichungstag der Anmeldung: 15.07.1998
(73) Patentinhaber: Wacker-Chemie GmbH, 81737 München (DE)
(72) Erfinder: BAUMANN, Frank, D-84561 Mehring (DE); DEUBZER, Bernward, D-84489 Burghausen (DE); GECK, Michael, D-84489 Burghausen (DE); SCHMIDT, Manfred, D-95488 Eckersdorf (DE)
(74) Vertreter: Fritz, Helmut, Dr.
(86) Internationale Anmeldenummer: EP9604209
(87) Internationale Veröffentlichungsnummer: WO9711984

(56) Entgegenhaltungen:
- EP-A- 0 638 604
- EP-A- 0 744 432
- US-A- 5 187 134

## Beschreibung

Die Erfindung betrifft aus einem einzigen Molekül bestehende monodisperse lösliche Organopolysiloxanpartikel, die chemisch gebundene Metallverbindungen aufweisen und deren Herstellung und Verwendung. Die Organopolysiloxanpartikel weisen einen mittleren Durchmesser von 5 bis 200 nm auf und befinden sich deshalb im mesoskopischen Größenbereich.

Bei homogen katalysierten Reaktionen mit bekannten Homogenkatalysatoren kann sich während eines kontinuierlichen Betriebes ein festes oder im Reaktionsmedium unlösliches Reaktionsprodukt an der Innenwand des Reaktors abscheiden. Dieser als Fouling bezeichnete Vorgang führt meist zu einem Betriebsabbruch.

Auf organischen Trägern, wie Polystyrol und anorganischen Trägern, wie Silica-Gel fixierte Komplexliganden und die damit herstellbaren Metallkomplexe sind zusammenfassend beschrieben in R.H. Grubbs, CHEMTECH, August 1977, S. 512 ff und D.D. Whitehurst, CHEMTECH, Januar 1980, S. 44ff. Ziel der Heterogenisierung der katalytisch aktiven Metallkomplexe ist dabei, die Nachteile der homogenen Katalyse zu umgehen und die Vorteile der homogenen Katalysatoren, nämlich hohe Selektivität und Aktivität, und die Vorteile der heterogenen Katalysatoren, nämlich leichte Abtrennung und Rückgewinnung des Katalysators aus einem flüssigen Reaktionsgemisch und kein Fouling bei festen Reaktionsprodukten, zu vereinigen.

Die bisher verwendeten anorganischen Trägermaterialien auf Siliciumdioxidbasis, wie pyrogene Kieselsäure oder gemahlene und gegebenenfalls thermisch modifizierte Silica-Gele weisen uneinheitliche Korngrößenverteilungen und Korngrößen von 0,001 mm bis 3 mm auf. Ein weiterer Nachteil dieser Trägermaterialien ist ihre unregelmäßige Partikel struktur und die Beschränkung auf geringe Metallkomplexdichte auf der Trägeroberfläche. Diese Partikel sind in organischen Lösungsmitteln nicht löslich.

Auf dem Sol-Gel-Verfahren beruhende Verfahren zur Herstellung von sphärischen Silica-Gelpartikeln mit einem Partikeldurchmesser von 0,01 mm bis 3 mm und einer relativ frei einstellbaren Belegungsdichte des Silica-Trägers mit der katalytisch aktiven Metallkomplexverbindung durch Kokondensation von amin- und phosphinfunktionalisierten Trialkoxysilanen, bevorzugt mit Tetraethoxysilan werden in DE-C 30 29 599 und DE-C 39 25 359, sowie in US-A 5,187,134 beschrieben. Die erhaltenen Katalysatorsysteme sind in allen Lösungsmitteln unlöslich.

Es bestand die Aufgabe, lösliche Organopolysiloxanpartikel bereitzustellen, die chemisch angebundene Metallverbindungen aufweisen und eine monodisperse Teilchengrößenverteilung innerhalb eines Größenbereichs von 5 bis 200 nm besitzen.

Die Erfindung betrifft aus einem einzigen Molekül bestehende vernetzte Organopolysiloxanpartikel, die chemisch angebundene Metallverbindungen aufweisen, einen mittleren Durchmesser von 5 bis 200 nm besitzen und die in mindestens einem der Lösungsmittel, die ausgewählt werden aus Dichlormethan, Pentan, Aceton, Ethanol und Wasser zu mindestens 1 Gew.% löslich sind, wobei mindestens 80 % der Partikel einen Durchmesser besitzen, der höchstens 30 % vom mittleren Durchmesser abweicht.

Die Organopolysiloxanpartikel weisen typischerweise mittlere Molmassen von mindestens 10⁵, insbesondere 5 x 10⁵, und vorzugsweise höchstens 10¹⁰ g/mol, insbesondere 10⁹ auf. Die mittleren Durchmesser der Organopolysiloxanpartikel betragen vorzugsweise mindestens 10 und höchstens 150 nm.
Vorzugsweise besitzen mindestens 80 % der Partikel einen Durchmesser, der höchstens 20 %, insbesondere höchstens 10 % vom mittleren Durchmesser abweicht.
Bei den Organopolysiloxanpartikeln handelt es sich vorzugsweise um sphärische Partikel.

Die Organopolysiloxanpartikel sind in Lösungsmitteln löslich und können deshalb wie homogene Katalysatoren eingesetzt werden, wobei aber der katalytisch aktive Komplex auf dem mesoskopischen Trägersystem immobilisiert ist. Vorzugsweise beträgt die Löslichkeit in einem Lösungsmittel mindestens 2 Gew.-%, insbesondere 10 Gew.-%. Das Lösungsmittel, in dem sich die Organopolysiloxanpartikel lösen, hängt vom Aufbau der Organopolysiloxanpartikel und insbesondere von den sich an der Oberfläche der Organopolysiloxanpartikel befindenden Gruppen ab. Für alle Organopolysiloxanpartikel gibt es ein geeignetes Lösungsmittel. Beispiele für solche Lösungsmittel sind Wasser; Alkohole, wie Methanol, Ethanol, n-Propanol, iso-Propanol; Ether, wie Dioxan, Tetrahydrofuran, Diethylether, Diethylenglycoldimethylether; chlorierte Kohlenwasserstoffe, wie Dichlormethan, Trichlormethan, Tetrachlormethan, 1,2-Dichlorethan, Trichlorethylen; Kohlenwasserstoffe, wie Pentan, n-Hexan, Cyclohexan, Hexan-Isomerengemische, Heptan, Oktan, Waschbenzin, Petrolether, Benzol, Toluol, Xylole; Ketone, wie Aceton, Methylethylketon, Methyl-isobutylketon; Dimethylformamid, Schwefelkohlenstoff und Nitrobenzol, oder Gemische dieser Lösungsmittel, sowie Monomere, wie Methylmethacrylat oder Styrol und Polymere, wie flüssige Organopolysiloxane.

Die Anzahl der chemisch angebundenen Metallverbindungen pro Organopolysiloxanpartikel beträgt mindestens 1, vorzugsweise mindestens 10, insbesondere mindestens 100, und vorzugsweise höchstens 10⁶, insbesondere höchstens 10⁵, besser höchstens 10⁴. Die Bestimmung der Anzahl der Metallverbindungen kann durch spektroskopische Methoden, wie UV-Vis erfolgen.

Die Metallverbindungen, die an den Organopolysiloxanpartikeln chemisch gebunden sind, werden vorzugsweise ausgewählt aus Metallverbindungen der II., III., IV., und V. Hauptgruppe und den Nebengruppen des periodischen Systems der Elemente, insbesondere Metallverbindungen der I., IV., VI., VII und VIII. Nebengruppe.

Vorzugsweise sind die Metallverbindungen an der Oberfläche der Organopolysiloxanpartikel koordinativ gebunden, es handelt sich dann um Komplexverbindungen, wobei das Metallatom einer Metallverbindung an einem oder mehreren Organopolysiloxanpartikeln gebunden sein kann. Ein Metallatom einer Metallverbindung kann am selben Organopolysiloxanpartikel über einen oder mehrere Liganden koordinativ gebunden sein. Die Liganden, an die die Metallverbindungen koordinativ gebunden sind, können direkt an einem Siliciumatom an der Oberfläche der Organopolysiloxanpartikel gebunden sein oder über einen Spacer, d.h einen zweiwertigen organischen Rest an einem Siliciumatom haften. Die Metallverbindung kann neben den über Liganden koordinativ gebundenenen Organopolysiloxanpartikeln noch niedermolekulare Liganden zur Absättigung noch gegebenenfalls vorhandener freier Koordinationsstellen und/oder ggf. Gegenionen zum Ladungsausgleich besitzen.

Besonders bevorzugte Organopolysiloxanpartikel weisen an der Oberfläche Einheiten auf, die ausgewählt werden aus Einheiten der allgemeinen Formeln

[AR₂SiO_{1/2}] **(1),**

[ARSiO_{2/2}] **(2)**

und

[ASiO_{3/2}] **(3),**

und die restlichen Einheiten der Organopolysiloxanpartikel bestehen aus
0,5 bis 80,0 Gew.-% Einheiten der allgemeinen Formel

[R₃SiO_{1/2}) **(4),**

0 bis 99,0 Gew.-% Einheiten der allgemeinen Formel

[R₂SiO_{2/2}] **(5),**

0 bis 99,5 Gew.-% Einheiten der allgemeinen Formel

[RSiO_{3/2}] **(6)**

und
0 bis 80,0 Gew.-% Einheiten der allgemeinen Formel

[SiO_{4/2}] **(7),**

wobei
- **A**: eine Ligandeneinheit der allgemeinen Formeln

spL' **(8)**

oder

spsp'L" **(9)**

ist, wobei mindestens eine Einheit der allgemeinen Formeln (8) oder (9) pro Organopolysiloxanpartikel in einem Komplex der allgemeinen Formeln

spL"ᵢMLₖ **(10)**

oder

spsp'L"ᵢMLₖ **(11)**

gebunden ist, und wobei
- **M**: ein Metall der I., IV., VI., VII. und VIII. Nebengruppe des periodischen Systems der Elemente,
- **L**: einen Komplexliganden in der Koordinationssphäre des Metalls **M**,
- **L'**: einen über einen Spacer **sp** an die Oberfläche eines Organopolysiloxanpartikels gebundenen Komplexliganden in der Koordinationssphäre des Metalls **M**,
- **L"**: einen über zwei Spacer **sp** und **sp'**an die Oberfläche eines Organopolysiloxanpartikels gebundenen Komplexliganden in der Koordinationssphäre des Metalls **M**,
- **sp** und **sp'**: gleiche oder verschiedene zweiwertige SiC-gebundene, gegebenenfalls substituierte C₀- bis C₁₈-Kohlenwasserstoffreste, welche durch zweibindige, beidseitig an Kohlenstoffatome gebundene Reste aus der Gruppe -O-, -COO-, -OOC-, -CONR²-, -NR²CO-, -CO- und-[SiR₂]ₗ- unterbrochen sein können,
- **R**: gleiche oder verschiedene einwertige SiC-gebundene, gegebenenfalls halogensubstituierte C₁- bis C₁₈-Alkylreste,
- **i**: Werte von 1 bis höchstens die Koordinationszahl des Metalls **M**,
- **k**: Werte von 0 bis höchstens die Koordinationszahl des Metalls **M** minus i und
- **l**: Werte von 1 bis 100 bedeuten.

Vorzugsweise besitzt der Komplexligand **L** ein Molekulargewicht von höchstens 500, insbesondere von höchstens 200 g/mol. Beispiele für Komplexliganden **L** sind Kohlenmonoxid, Stickoxid, Trialkylphosphine, Alkylarylphosphine, Triphenylphosphin, Tetraphenyldiphosphine verbrückt durch Kohlenwasserstoffreste mit 1 bis 6 Kohlenstoffatome, Phosphit, Triphenylarsin, Trialkoxyphosphine, sulfonierte Phosphane, carboxylierte Phosphane, Phosphane mit quarternisierten Aminoalkyl- und Aminoarylsubstituenten, Phosphane mit Hydroxyalkyl- und Polyethersubstituenten, Phosphinoalkylphosphoniumsalze, ein sekundäres oder tertiäres Alkylamin mit 1 bis 10 C-Atomen enthaltenden linearen oder verzweigten Alkylgruppen, sekundäre oder tertiäre Diamine, die mit 1 bis 6 C-Atomen verbrückt sein können, Benzylamin, Dialkylsulfid, Olefine, Diolefine, cyclische Olefine mit 4 bis 30 C-Atome, cyclische Diolefine, wie Cyclooctadien mit 4 bis 30 C-Atomen, cyclische aromatischen Verbindungen, wie der Cyclopentadienylring mit 5 bis 30 C-Atomen, Alkine, Nitril, Isonitril, Cyanat, Isocyanat und Lösungsmittel wie Wasser, Ether und Alkohole.

Ein gegebenenfalls erforderlicher Ladungsausgleich erfolgt bei allen erfindungsgemäßen Organopolysiloxanpartikeln mit anorganischen oder organischen Anionen, wie Chlorid-, Bromid-, Iodid-, Nitrat-, Sulfat-, Phosphat-, Acetylacetonat-, Acetat-, Trifluoracetat-, Trichloracetat-, Propionat-, Methylat, Ethylat-, Propylat-, Butylat-, Phenylat-, Methylaluminiumoxylat-, Perchlorat-, Tetraphenylborat-, Hexafluorophosphat-, Methyl-, Ethyl-, Propyl-, Butyl-, Phenyl- oder Perfluorophenylion, gegebenenfalls unter vollständigem oder partiellem Ersatz solcher Anionen durch Hydridionen.

Beispiele für Komplexliganden **L**' sind lineare und cyclische Alkenylreste mit bis zu 30 C-Atomen, wie der Vinyl-, Allyl-, n-5-Hexenyl-, 4-Vinylcyclohexyl- und der 3-Norbornenylrest; lineare und cyclische Alkendienylreste mit bis zu 30 C-Atomen, wie der Butadienyl-, Cyclopentadienyl- und

Cyclooctadienylrest; Arylreste mit bis zu 30 C-Atomen, wie der Fluorenyl- und Indenylrest; heterocyclische Reste mit bis zu 30 C-Atomen, die ein oder mehrere gleiche oder verschiedene Heteroatome, wie Stickstoff-, Sauerstoff- oder Schwefelatome enthalten, wie Pyridinderivate, Bipyridinderivate, Imidazolderivate, Thiophenderivate und Furanderivate; Mercaptoalkylreste mit 2 bis 20 C-Atomen, Thioalkylreste mit 2 bis 20 C-Atomen, Cyanoalkylreste mit 2 bis 20 C-Atomen, sekundäre und tertiäre Aminoalkylreste mit 2 bis 30 C-Atomen, sekundäre und tertiäre Diaminalkylreste mit 2 bis 30 C-Atomen, sekundäre und tertiäre Aminoarylreste mit 6 bis 30 C-Atomen, Acyloxyalkylreste mit 2 bis 30 C-Atomen, Oxyalkylreste mit bis zu 30 C-Atomen, Phosphinalkylreste mit bis zu 30 C-Atomen, Diphosphinalkylreste mit bis zu 30 C-Atomen, Phosphinarylreste mit 6 bis 30 C-Atomen, Phosphinalkylarylreste mit 6 bis 30 C-Atomen, Harnstoffderivate mit bis zu 30 C-Atomen, Thioharnstoffderivate mit bis zu 30 C-Atomen und 1,3-Diketone mit bis zu 30 C-Atomen.

Bevorzugte Komplexliganden **L**' sind unsubstituierte und substituierte C₂- bis C₆-Alkylreste, insbesondere der 3-Mercaptoalkylpropyl-, 3-Aminopropyl-, (2-Aminoethyl)-3-aminopropyl-, (3-Dialkylphosphino)- und (3-Alkylarylphosphino)-propylrest; C₂- bis C₈- Alkenylreste, insbesondere der Vinyl- und Allylrest; C₄-bis C₂₀-Alkyldienylreste, insbesondere der Cyclopentadienyl- und Cycooctadienylrest; C₆- bis C₂₀- Arylreste, insbesondere der Indenyl- und Fluorenylrest sowie über -CR₂²-, -(CH₂)ₙ- oder -SiR₂2- verbrückte Indenyl-cyclopentadienyl-, Fluorenylcyclopentadienyl-, Indenyl-Indenyl- und Fluorenylfluorenylreste, wobei R² und n die vorstehenden Bedeutungen aufweisen; C₄- bis C10-Heterocyclenreste, insbesondere der Pyridin- und Bipyridinrest.

Beispiele für die zweiwertigen Komplexliganden **L"** sind die Gruppen -N=N-, -N=N-NR²-, -NR²-, -NR²-CO-NR²-, -NR²-CS-NR²-, -[NR²-(CH₂)ₙ]ₘ-, -O-O-, -CO-CO-, -CO-CH₂-CO-, -[O-(CH₂)ₙ]ₘ-, -Sx-, -PR²-, [-PR²-(CH₂)ₙ]ₘ-, bei denen
- R²: ein Wasserstoffatom oder einen Rest R,
- m: die Werte 1,2,3 oder 4,
- n: die Werte 1,2,3 oder 4 und
- x: die Werte 1,2,3,4 bedeuten.

Weitere Beispiele für die Komplexliganden **L"** sind die für Komplexliganden **L'**aufgeführten Beispiele, die dann zwei Bindungsstellen für die Spacer **sp** und **sp'**aufweisen.

Bevorzugte zweiwertige Komplexliganden **L"** sind -NR²-, -[NR²-(CH₂)₂]₂-, -PR²- und -[PR²-(CH₂)₂]₂-, insbesondere -NR²- und -PR²-, bei denen **R**^{**2**} einen unsubstituierten C₁- bis C₆-Alkylrest bedeutet.

Die Kohlenstoffatome an den Komplexliganden **L, L'** und **L"** können substituiert sein, beispielsweise durch Halogenatome, wie Fluor-, Chlor-, oder Bromatome und Cyanogruppen.

Die Komplexliganden **L, L'** und **L"** können sterisch anspruchsvoll und/oder chiral sein, dadurch wird auch der Komplex sterisch anspruchsvoll und ist beispielsweise für stereoselektive Reaktionen einsetzbar.

Beispiele für die zweiwertigen Reste **sp** und **sp'** sind gesättigte Alkylenreste wie der Methylen- und Ethylenrest, sowie Propylen-, Butylen-, Pentylen-, Hexylen-, Cyclohexylen- und Octadecylenreste oder ungesättigte Alkylen- oder Arylenreste, wie der Hexenylenrest und Phenylenreste und Polyoxyalkylenreste.

Bevorzugte Reste **sp** und **sp'** sind gesättigte Alkylenreste mit 1 bis 10 C-Atomen, insbesondere der Methylen-, Ethylen- und Propylenrest.

Beispiele für unsubstituierte Reste **R** sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentyl-rest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, Octadecylreste, wie der n-Octadecylrest; Cycloalkylreste, wie Cyclopentyl-, Cyclohexyl-, 4-Ethylcyclohexyl-, Cycloheptylreste, Norbornylreste und Methylcyclohexylreste. Beispiele für halogensubstituierte Alkylreste als Rest R sind der Chlormethyl-, 3-Chlorpropyl-, 3-Brompropyl-, 3,3,3-Trifluorpropyl- und 5,5,5,4,4,3,3-Heptafluorpentylrest.

Bei dem Rest **R** handelt es sich bevorzugt um unsubstituierte C₁- bis C₆-Alkylreste, Phenylreste und Wasserstoff, insbesondere um den Methylrest.

Vorzugsweise bedeuten unabhängig voneinander
- **i**: die Werte 1, 2 oder 3, insbesondere 1,
- **k**: die Werte 1, 2, 3 oder 4 und
- **l**: Werte von 2 bis 20.

Vorzugsweise enthalten die Organopolysiloxanpartikel mindestens insgesamt 0,1 Gew.-% an Einheiten der allgemeinen Formel (1) bis (3),
und die restlichen Einheiten der Organopolysiloxanpartikel bestehen aus
1 bis 80,0 Gew.-% Einheiten der allgemeinen Formel (4),
0 bis 98,0 Gew.-% Einheiten der allgemeinen Formel (5),
0 bis 99,0 Gew.-% Einheiten der allgemeinen Formel (6) und
0 bis 50,0 Gew.-% Einheiten der allgemeinen Formel (7) mit der Maßgabe, daß die Summe der Einheiten der allgemeinen Formeln (6) und (7) mindestens 1 Gew.-% beträgt.

Organopolysiloxanpartikel, die mindestens zu 80 Mol-% aus Einheiten der allgemeinen Formeln (2) und (5) aufgebaut sind, haben elastomere Eigenschaften. Diese Partikel sind in den vorstehenden organischen Lösungsmitteln, insbesondere in Toluol, Tetrahydrofuran, Dioxan, Petrolether, chlorierte Kohlenwasserstoffen wie H₂CCl₂ und HCCl₃ quellbar. In diesen Organopolysiloxanpartikeln sind in aufgequollenem Zustand auch im Innern Metallkomplexe von beispielsweise umzusetzenden Verbindungen erreichbar.

Ein weiterer Gegenstand der Erfindung ist ein Verfahren zur Herstellung der vernetzten Organopolysiloxanpartikel, die chemisch gebundene Metallverbindungen aufweisen, bei dem in einem ersten Schritt durch Zudosieren von Silanen der allgemeinen Formel (12)

RₐSi(OR²)₄₋ₐ **(12),**

und gegebenenfalls siliciumorganischen Verbindungen, die aufgebaut sind aus Einheiten der allgemeinen Formel (13)

R_{b}(R²O)_{c}SiO_{(4-b-c)/2} **(13),**

zu einer bewegten Mischung aus Emulgator und Wasser eine kolloidale Suspension von Organopolysiloxanpartikeln hergestellt wird,
in einem zweiten Schritt die kolloidale Suspension mit Silanen der allgemeinen Formel (14)

AR_{d}Si(OR²)_{4-d} **(14),**

und/oder siliciumorganischen Verbindungen, die aufgebaut sind aus Einheiten der allgemeinen Formel (15)

AₑR_{f}(R²O)_{g}SiO_{(4-e-f-g)/2} **(15),**

versetzt wird,
mit der Maßgabe, daß die Verbindungen, die aufgebaut sind aus Einheiten der allgemeinen Formel (15), mindestens einen Rest A und einen Rest (R²O) aufweisen,
und in einem dritten Schritt die kolloidale Suspension mit siliciumorganischer Verbindung der allgemeinen Formel (16)

(R³R⁴ ₂Si)ₕX **(16),**

versetzt wird, mit der Maßgabe, daß die siliciumorganischen Verbindungen der allgemeinen Formel (16) wasserlöslich sind oder in Wasser zu einer wasserlöslichen Verbindung hydrolysieren,
wobei
- **R**^{**2**} und **R**^{**4**}: die Bedeutungen von R,
- **R**^{**3**}: die Bedeutungen von A oder R,
- **X**: für die Bedeutungen **h** = 1, -OR⁵,
-ONR⁵₂ oder -OOCR⁵ und
für die Bedeutungen **h** = 2 -O- oder -S-,
- **R**^{**5**}: die Bedeutungen von **R**,
- **a**: die Werte 0, 1, 2 oder 3,
- **b** und **c**: jeweils unabhängig voneinander die Werte 0, 1, 2, 3 oder 4,
- **d**: die Werte 0, 1, oder 2,
- **e, f** und **g**: jeweils unabhängig voneinander die Werte 0, 1, 2 oder 3,
- **h**: die Werte 1 oder 2 bedeuten und
- **R** und **A**: die vorstehenden Bedeutungen aufweisen.

Die interpartikuläre Kondensation der Organopolysiloxanpartikel wird verhindert, indem die nach dem ersten und zweiten Schritt verbliebenen, kondensationsfähigen Gruppen mit ausschließlich monofunktionelle Triorganosilylgruppen enthaltenden siliciumorganischen Verbindungen abgesättigt werden.

Vorzugsweise werden bei der Hydrolyse- oder Kondensationsreaktion der siliciumorganischen Verbindungen der allgemeinen Formel (16) keine Nebenprodukte, wie Salzsäure oder Ammoniak gebildet, die die Ionenstärke des wäßrigen kolloidalen Systems wesentlich vergrößern.

Besonders bevorzugt werden eingesetzt als siliziumorganische Verbindungen der allgemeinen Formel (16) Trialkylalkoxysilane, wie Trimethylmethoxysilan und Trimethylethoxysilan, vorzugsweise bedeuten **R**^{**2**} und **R**^{**3**} darin Methylgruppen; Dimethylalkenalkoxysilane, wobei **R**^{**2**} und **R**^{**3**} vorzugsweise Allyl- oder Vinylreste bedeuten; ebenso können **R**^{**2**} und **R**^{**3**} auch cyclische Alkene oder Alkadiene bedeuten. Insbesondere bevorzugte Alkoxyreste sind der Methoxy- und Ethoxyrest. Weiterhin bevorzugt sind Hexaalkyldisiloxane, wie Hexamethyldisiloxan. Besonders bevorzugt werden im dritten Schritt Trimethylmethoxysilan und Trimethylethoxysilan eingesetzt.

Die Isolierung der Organopolysiloxanpartikel aus den kolloidalen Suspensionen nach Beendigung des dritten Reaktionsschrittes kann nach bekannten Verfahren erfolgen, beispielsweise durch Brechen der Dispersion mittels Salz zugabe oder durch Zugabe von polaren Lösungsmitteln.

Organopolysiloxanpartikel, die in der Summe mehr als 15 Gew.-% an Einheiten der allgemeinen Formeln (6) und (7) enthalten, werden vorzugsweise nach Isolierung im Anschluß an den dritten Schritt in einem vierten Reaktionsschritt in einem aprotischen Lösungsmittel mit siliciumorganischer Verbindung der allgemeinen Formeln (17)

(R⁶R⁷ ₂Si)ᵢY **(17),**

oder (18) 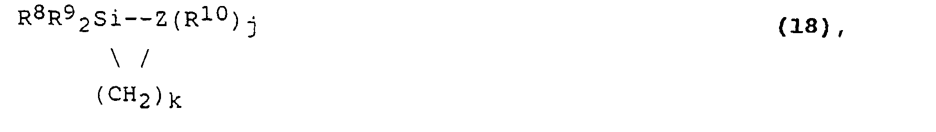 versetzt, wobei
- **R**^{**6**} und **R**^{**8**}: die Bedeutungen von **A** oder **R**,
- **R**^{**7**}**, R**^{**9**} und **R**^{**10**}: die Bedeutungen von **R**,
- **Y**: für die Bedeutung **i** = 1, Halogenatome,
-OR⁶, NR⁶₂, -ONR⁶₂ oder -OOCR⁶ und
für die Bedeutung **i** = 2 -O-, =N(R⁶) oder -S-,
- **Z**: für die Bedeutung **j** = 1 -N- und
für die Bedeutung **j** = 0 -O- oder -S-,
- **i**: die Werte 1 oder 2,
- **j**: die Werte 0 oder 1,
- **k**: die Werte 1 bis 30 bedeuten und
- **R** und **A**: die vorstehenden Bedeutungen aufweisen.

Besonders bevorzugt werden als siliziumorganische Verbindungen der allgemeinen Formeln (17) und (18) in diesem vierten Reaktionsschritt Alkylchlorsilane, wie Trimethylchlorsilan, Dimethylchlorsilan, Vinyldimethylchlorsilan, Hexamethyldisilazan, 1,3-Divinyl-1,1,3,3-tetramethyldisilazan, oder Gemische aus Disilazanen bzw. Chlorsilanen eingesetzt.

Die eingesetzten Mengen an Verbindungen der allgemeinen Formeln (12) bis (18) werden so gewählt, daß die gewünschten Organopolysiloxanpartikel erhalten werden. Die eingesetzten Mengen an Verbindungen der allgemeinen Formeln (12) und (13) werden im ersten Reaktionsschritt nahezu quantitativ eingebaut und steuern den Vernetzungsgrad der Organopolysiloxanpartikel in wäßriger Suspension.

Durch die im zweiten Reaktionsschritt eingesetzte Menge an Verbindungen der allgemeinen Formeln (14) und (15) wird bevorzugt die Metallverbindungsdichte an der Oberfläche der Organopolysiloxanpartikel gesteuert. Vorzugsweise werden 0,001 bis 0,5, insbesondere 0,01 bis 0,1 Gewichtsteile Verbindungen der allgemeinen Formeln (14) und (15) pro Gewichtsteil an Verbindungen der allgemeinen Formeln (12) und (13) eingesetzt.

Die im dritten und gegebenenfalls im vierten Reaktionsschritt eingesetzten Verbindungen der allgemeinen Formeln (16) bis (18) werden jeweils im überschuß eingesetzt und somit nicht vollständig in die Organopolysiloxanpartikel eingebaut. Vorzugsweise werden 0,01 bis 10, insbesondere 0,1 bis 1 Gewichtsteile Verbindungen der allgemeinen Formeln (16) im dritten Reaktionsschritt, bzw. der Summe der Verbindungen der allgemeinen Formeln (16) bis (18) im dritten und vierten Reaktionsschritt, pro Gewichtsteil an Verbindungen der allgemeinen Formeln (12) und (13) eingesetzt.

Wenn ein vierter Reaktionsschritt durchgeführt wird beträgt das Verhältnis der im dritten Reaktionsschritt eingesetzten Menge der Verbindungen der allgemeinen Formeln (16) : der im vierten Reaktionsschritt eingesetzten Menge der Verbindungen der allgemeinen Formeln (16) bis (18) vorzugsweise 1 : 10 bis 2 : 1, insbesondere 1 : 5 bis 1 : 1.

Bei dem Rest R in den im Verfahren eingesetzten Verbindungen handelt es sich bevorzugt um unsubstituierte C₁- bis C₆-Alkylreste und den Phenylrest, wobei Methyl-, Ethyl- und Propylreste besonders bevorzugt sind.

Die Ligandeneinheiten A in den allgemeinen Formeln (14) bis (18) können bereits in einen Komplex der allgemeinen Formeln (10) oder (11) gebunden sein oder nach dem zweiten, dritten oder vierten Schritt durch Umsetzung einer Ligandeneinheit mit einer Metallverbindung hergestellt werden.

Beispiele für geeignete Metallverbindungen sind FeB₃, FeB₂, CoB₃, CoB₂, NiB₂, RuB₃, RuB₂, RhB₃, RhB₂, RhB, Rh(dien)B, RhB(CO), PdB₄, PdB₂, OsB₃, IrB₃, IrB, Ir(dien)B, IrB(CO), PtB₄ und PtB₂, wobei B ein Chlor-, Brom-, Jod-, Wasserstoffatom oder Acetylacetonat, Acetat, 0.5 SO₄, NO₃ oder CN und dien Cyclooctadien oder Norbornadien bedeutet.

Als Metallverbindungen sind auch Komplexverbindungen wie FeB₃L₃, FeB₂L₄, CoB₃L₂, CoB₃L₃, CoB₂L₃, CoB₂L₄, NiB₂L₂, NiL₄, RuB₃L₃, RhB₃L₃, RhB₂L₃, RhBL₃, RhL⁴⁺B⁻, PdB₄L₂, PdB₂L₂, PdL₄, OsB₃L₃, IrB₃L₃, IrBL₃, PtB₄L₂, PtB₂L₂ und PtL₄ einsetzbar, wobei B und L die vorstehenden Bedeutungen aufweisen.

Im Herstellungsverfahren besonders geeignete Emulgatoren sind:
Alkylsulfate, z.B. mit einer Kettenlänge von 8-18 C-Atomen, Aryl- und Alkylethersulfate mit 8-18 C-Atomen im hydrophoben Rest und 1-40 Ethylenoxid(EO)- bzw. Propylenoxid(PO)-Einheiten;
Sulfonate, z.B. Alkylsulfonate mit 8-18 C-Atomen, Alkylarylsulfonate mit 8-18 C-Atomen, Ester und Halbester der Sulfobernsteinsäure mit einwertigen Alkoholen oder Alkylphenolen mit 4-15 C-Atomen; ggf. können diese Alkohole oder Alkylphenole auch mit 1-40 EO-Einheiten ethoxyliert sein;
Alkali- und Ammoniumsalze von Carbonsäuren mit 8-20 C-Atomen im Alkyl-, Aryl-, Alkaryl- oder Aralkylrest;
Phosphorsäureteilester und deren Alkali- und Ammoniumsalze, z. B. Alkyl- und Alkarylphosphate mit 8-20 C-Atomen im organischen Rest, Alkylether- bzw. Alkaryletherphosphate mit 8-20 C-Atomen im Alkyl- bzw. Alkarylrest und 1-40 EO-Einheiten;
Alkylpolyglykolether mit 2-40 EO-Einheiten und Alkylresten von 4-20 C-Atomen;
Alkylarylpolyglykolether mit 2-40 EO-Einheiten und 8-20 C-Atomen in den Alkyl- und Arylresten;
Ethylenoxid/Propylenoxid(EO/PO)-Blockcopolymere mit 8-40 EO-bzw. PO-Einheiten;
Fettsäurepolyglykolester mit 6-24 C-Atomen und 2-40 EO-Einheiten;
Alkylpolyglykoside, Naturstoffe und deren Derivate, wie Lecithin, Lanolin, Saponine, Cellulose; Cellulosealkylether und Carboxyalkylcellulosen, deren Alkylgruppen jeweils bis zu 4 Kohlenstoffatome besitzen;
polare Gruppen enthaltende lineare Organo(poly)siloxane mit Alkoxygruppen mit bis zu 24 C-Atomen und/oder bis zu 40 EO-und/oder PO-Gruppen;
Salze von primären, sekundären und tertiären Fettaminen mit 8-24 C-Atomen mit Essigsäure, Schwefelsäure, Salzsäure und Phosphorsäuren;
Quarternäre Ammoniumsalze wie Halogenide, Sulfate, Phosphate, Acetate oder Hydroxide, deren Alkylgruppen unabhängig voneinander 1-24 C-Atome besitzen; gegebenenfalls können die Alkyl- oder Alkaryl- oder Aralkylgruppen der quarternären Ammoniumverbindungen auch teilweise ethoxyliert sein (1-40 EO-Einheiten);
Alkylpyridinium-, Alkylimidazolinium- und Alkyloxazoliniumsalze, deren Alkylkette bis zu 18 C-Atome besitzt, in Form ihrer Halogenide, Sulfate, Phosphate oder Acetate.

Bevorzugt sind aliphatisch substituierte Benzolsulfonsäuren und deren Salze sowie gegebenenfalls teilethoxylierte quarternäre Ammoniumhalogenide und -hydroxide. Besonders bevorzugt sind Dodecylbenzolsulfonsäure und Benzyldimethyl-{2-[2-(p-1,1,3,3-tetramethylbutyl-phenoxy)-ethoxy]-ethyl}-ammoniumchlorid (Benzethoniumchlorid).

Die einzusetzende Menge an Emulgator beträgt von 0,5 - 50 Gew.-%, vorzugsweise von 1,0 - 30 Gew.-%, jeweils bezogen auf die insgesamt eingesetzte Menge an siliziumorganischen Ausgangsverbindungen des ersten, zweiten und dritten Reaktionsschrittes. Die siliziumorganischen Ausgangsverbindungen der allgemeinen Formeln (12) und (13) werden während des ersten Reaktionsschrittes bevorzugt dosiert zugegeben. Vorzugsweise werden alle Ausgangskomponenten der allgemeinen Formeln (12) und (13) vor der Dosierung während des ersten Reaktionsschritts im gewünschten Verhältnis gemischt; um eine homogene Mischung zu erhalten, werden gegebenenfalls zusätzlich 0,1 - 30 Gew.-% bezogen auf die Summe der Ausgangskomponenten der allgemeinen Formeln (12) und (13), Alkanol der Formel R¹¹OH, bei dem R¹¹ ein Alkylrest mit 1 bis 5 Kohlenstoffatomen ist, als Löslichkeitsvermittler zugesetzt, wobei die Alkanole Methanol und Ethanol besonders bevorzugt sind.

Als aprotisches organisches Lösungsmittel werden im vierten Schritt vorzugsweise die vorstehend beschriebenen Ether, Kohlenwasserstoffe, Ketone und Organopolysiloxane, insbesondere Tetrahydrofuran, Cyclohexan, Methylcyclohexan oder Toluol verwendet. Die Umsetzung sowohl im ersten (Emulsionspolykondensation/-polymerisation) als auch im zweiten und dritten Reaktionsschritt wird vorzugsweise bei 5 - 95 °C, insbesondere bei 10 - 85 °C und besonders bevorzugt bei 10 - 40 °C durchgeführt. Der pH-Wert beträgt jeweils von 1 - 12, vorzugsweise von 1 - 4 oder von 7 - 11, in Abhängigkeit von der Säure-/Basestabilität der Reste **R**, **R**^{**1**}, **R**^{**2**}, **R**^{**3**}, **R**^{**4**} und **R**^{**5**} der Ausgangsverbindungen (12) bis (16).

Bei der Herstellung der kolloidalen Suspensionen während des ersten Reaktionsschrittes ist es für die Stabilität der Emulsion vorteilhaft, nach Ende der Dosierung der siliziumorganischen Ausgangsverbindungen der allgemeinen Formeln (12) und (13) noch 1 bis 24 Stunden nachzurühren. Das bei der Hydrolyse freigesetzte Alkanol kann durch Destillation, ggf. unter reduziertem Druck, entfernt werden, was aber nicht bevorzugt ist. Der Festgehalt der nach dem ersten Schritt hergestellten kolloidalen Suspension sollte vorzugsweise maximal 25 Gew.-% betragen, da sonst ein hoher Anstieg der Viskosität die weitere Umsetzung erschwert. Bei der Umsetzung der kolloidalen Suspension mit siliziumorganischer Verbindung der allgemeinen Formeln (14) und (15) im zweiten Reaktionsschritt ist es zur Erzielung einer möglichst vollständigen Reaktion ebenfalls vorteilhaft, nach Ende der Zugabe von Verbindungen der allgemeinen Formeln (12) und (13) noch 1 - 48 Stunden nachzurühren.

Die Umsetzung mit siliziumorganischer Verbindung der allgemeinen Formeln (17) und (18) im vierten Reaktionsschritt wird vorzugsweise bei 5 - 95 °C, insbesondere bei 10 - 85 °C und besonders bevorzugt bei 10 - 40 °C durchgeführt. Zur Erzielung einer möglichst vollständigen Reaktion ist es wiederum vorteilhaft, nach Ende der Zugabe der Verbindung der allgemeinen Formeln (12) und (13) noch 1 - 24 Stunden nachzurühren.

Wenn im zweiten, dritten oder vierten Schritt Siliciumverbindungen eingesetzt werden, die mehrere hydrolysierbare oder kondensierbare Stellen aufweisen, können zusätzliche Schalen an der Oberfläche der Organopolysiloxanpartikel gebildet werden.

Bei der Herstellung von Organopolysiloxanpartikeln aus mindestens 80 Mol-% Silanen der allgemeinen Formeln (12), in der a den Wert 2 hat und (14), in der d den Wert 1 hat können erster und zweiter Schritt zu einem Schritt zusammengefaßt werden. In diesem Schritt wird durch gemeinsames Zudosieren von Verbindungen der allgemeinen Formeln (12) bis (15) zu einer bewegten Mischung aus Emulgator und Wasser eine kolloidale Suspension von Organopolysiloxanpartikeln hergestellt. Da diese Organopolysiloxanpartikel quellbare Elastomere sind sind die Metallverbindungen im Innern der Partikel leicht, beispielsweise für weitere katalytische Reaktionen, zugänglich.

Wenn bei der Herstellung der Organopolysiloxanpartikel in den Verbindungen der allgemeinen Formeln (14) bis (18) keine Metallverbindung vorhanden ist, werden die Partikel in einem weiteren Schritt mit einer Metallverbindung zu Organopolysiloxanpartikeln, die chemisch angebundene Metallverbindungen aufweisen, umgesetzt. Die eingesetzte Metallverbindung kann ein Metallsalz oder eine Metallkomplexverbindung sein. Die sich an den Organopolysiloxanpartikeln befindlichen Liganden, wie Amino-, Mercapto- und Alkylhalogenidgruppen können vor der Umsetzung mit Metallverbindungen auch weiter chemisch modifiziert werden.

Wenn bei der Herstellung der in organischen Lösungsmitteln löslichen Organopolysiloxanpartikel sich reaktive Gruppen, wie Alkenyl- oder Si-H Gruppen am Partikel befinden, können in einem weiteren Schritt Liganden aufweisende Siliciumverbindungen an den reaktiven Gruppen gebunden werden. Beispielsweise können an die Alkenylgruppen Si-H Gruppen und Liganden aufweisende Siliciumverbindungen mit Hilfe eines Hydrosilylierungskatalysators gebunden werden. Beispielsweise können an die Si-H Gruppen Alkenylgruppen und Liganden aufweisende Siliciumverbindungen mit Hilfe eines Hydrosilylierungskatalysators gebunden werden.

Wenn sich bei der Herstellung der Organopolysiloxanpartikel nach dem dritten Schritt sich noch Silanolgruppen an der Oberfläche befinden, können in einem weiteren Schritt mit Metallhalogenidverbindungen unter Abspaltung von Halogenwasserstoff Komplexe gebildet werden.

Zur strukturellen Charakterisierung der Organopolysiloxanpartikel, die chemisch angebundene Metallverbindungen aufweisen, eignet sich besonders die statische und dynamische Lichtstreuung. Statische und dynamische Lichtstreuung sind dem Fachmann bekannte, etablierte Methoden in der makromolekularen Chemie und Kolloidchemie zur Charakterisierung disperser Partikel. In der statischen Lichtstreuung mittelt man die Streuintensität bei verschiedenen Winkeln über ein genügend langes Zeitintervall und erhält Aussagen über die statischen Eigenschaften der Makromoleküle, wie dem Gewichtsmittel der Molmasse M_{w}, dem z-Mittel des Quadrats des Trägheitsradius <R_{g}²>_{z}, und dem zweiten Virialkoeffizienten A₂, der die intra- und intermolekularen thermodynamischen Wechselwirkungen der dispergierten Partikel mit dem Lösungsmittel beschreibt. Im Gegensatz zur statischen wird bei der dynamischen Lichtstreuung die Fluktuation der Streulichtintensität als Funktion der Zeit beobachtet. Dies führt zu Aussagen über das dynamische Verhalten der untersuchten Moleküle. Gemessen werden das z-Mittel des Diffusionskoefizienten D_{z} und damit über das Stokes-Einstein-Gesetz der hydrodynamische Radius Rₕ und der Koeffizient kd, der die Konzentrationsabhängigkeit des Diffusionskoeffizienten beschreibt. Aus der Winkel abhängigkeit des Streulichts kann man die Teilchenform ermitteln und gegebenenfalls vorhandene Strukturierungen in Lösung aufklären. Die simultane statische und dynamische Lichtstreumessung ermöglicht es, mit einem einzigen Experiment die oben genannten Aussagen über das untersuchte System zu treffen und somit Informationen z.B. über Partikelgröße, -dispersität, und -form, sowie über Molekulargewicht und Dichte zu erhalten. Dies ist beispielsweise in M. Schmidt, Simultaneous Static and Dynamic Light Scattering: Applications to Polymer Structure Analysis, in: Dynamic Light Scattering: The Method and some Applications; Brown, W. (Hrsg.); Oxford University Press, Oxford, UK, 372-406 (1993) beschrieben.

Der Quotient aus Trägheits- und hydrodynamischem Radius, das sogenannte ρ-Verhältnis, liefert strukturelle Informationen über die Teilchenform, wie harte Kugel, Hohlkugel, Knäuel, Stäbchen oder Sternpolymer. Für die Teilchenform "harte Kugel" beträgt das theoretische ρ-Verhältnis 0,775; die bei den bevorzugten Organopolysiloxanpartikeln gemessenen Werte liegen von 0,775 bis höchstens ρ=1,0 . Die bevorzugten Organopolysiloxanpartikel sind deshalb spärisch.

Der Größenbereich der Organopolysiloxanpartikel stellt den Grenzbereich zwischen großen Molekülen, Oligomeren und Dendrimeren einerseits und kleinen Festkörpern andererseits dar, entspricht somit einer Nahtstelle zwischen Festkörper und Molekül. Zum einen sind kollektive Festkörpereigenschaften noch nicht ausgebildet, zum anderen ist molekulares Verhalten nicht mehr oder nur noch ansatzweise zu beobachten. Beispiele für partikuläre Strukturen dieser Größenordnung mit nahezu fixierter Konformation sind Mikrogele. Nach Antonietti (Angew. Chemie 100 (1988) 1813-1817) werden aus wäßrigen kolloidalen Systemen erhaltene Mikrogele mit Partikeldurchmessern im mesoskopischen Größenbereich und Molmassen von 10⁶ bis 10¹¹ g/Mol als "Typ B"-Mikrogele bezeichnet.

Die Organopolysiloxanpartikel, welche chemisch gebundene Metallverbindungen aufweisen sind als Katalysatoren, insbesondere homogene Katalysatoren, wobei aber der katalytisch aktive Komplex auf dem löslichen, mesoskopischen Trägerteilchen immobilisiert bzw. heterogenisiert ist in vielen Reaktionen und Reaktionssystemen einsetzbar. Beispiele für Katalysatoranwendungen sind Hydroformylierung, Hydrierung von Mehrfachbindungen, Hydrosilylierung, Strahlungsinduzierte katalytische Reaktionen und Olefinpolymerisation.

In den nachfolgenden Beispielen sind, falls jeweils nicht anders angegeben,
a) alle Mengenangaben auf das Gewicht bezogen;
b) alle Drücke 0,10 MPa (abs.) ;
c) alle Temperaturen 20° C .

### Beispiele:

Die Partikelgröße wird mittels Lichtstreuung gemessen:

Statische und dynamische Lichtstreuung werden mit einer Anlage gemessen, die u. a. aus einem Stabilite^{R} TM 2060-lls Kr-Laser von Spectra-Physics, einem Goniometer Sp-86 von ALV und einem ALV-3000 Digital Strukturator/Korrelator besteht. Der Krypton-Ionenlaser arbeitet mit einer Wellenlänge von 647,1 nm.

Probenpräparation: Die Proben der Organopolysiloxanpartikel in einem entsprechenden Lösungsmittel werden dreimal durch Millex^{R} TM-FGS-Filter (0.2 µm Porengröße) von Millipore filtriert. Der Konzentrationsbereich beträgt jeweils 0,5 - 2 g/l. Die Meßtemperatur bei den Lichtstreuexperimenten beträgt 20°C. Die dynamischen Lichtstreumessungen werden winkelabhängig von 50° bis 130° in 20°-Schritten durchgeführt, die Korrelationsfunktionen werden mit dem Simplex-Algorithmus ausgewertet. Beim statischen Lichtstreuexperiment wird die Winkelabhängigkeit des Streulichts von 30° bis 140° in 5°-Schritten gemessen.

### Synthese der Grunddispersion I

Zu einer Vorlage aus 500g Wasser und 2g Dodecylbenzolsulfonsäure werden bei Raumtemperatur im Verlauf von 90 Minuten 100g Methyltrimethoxysilan zudosiert. Die Reaktionslösung wird anschließend für 3 Stunden gerührt.

### Synthese der Grunddispersion II

Zu einer Vorlage aus 500g Wasser und 5g Dodecylbenzolsulfonsäure wird bei 80°C im Verlauf von 90 Minuten 100g eines Gemisches aus 95g Dimethyldimethoxysilan und 5g Methyltrimethoxysilan zudosiert. Die Reaktionslösung wird anschließend bei 80°C für 3 Stunden gerührt.

### Beispiel 1

### (Aminofunktionalisierte Organopolysiloxanpartikel)

Zu 100g der Grunddispersion I werden jeweils 5g eines in Tabelle 1 aufgeführten Trimethoxyalkylamins unter Rühren bei Raumtemperatur zudosiert. Die Dispersionen werden drei Stunden bei Raumtemperatur nachgerührt.

Je 52,5 g der erhaltenen aminofunktionalisierten Grunddispersionen werden mit 3g Trimethylmethoxysilan bei Raumtemperatur versetzt. Nach weiteren 15 Stunden Rühren werden die Dispersionen mit 100 ml Methanol gebrochen und abfiltriert. Nach dreimaligem Waschen mit je 50 ml Methanol wird der Feststoff in Toluol aufgenommen, mit 4g Hexamethyldisilazan versetzt und 10 Stunden bei Raumtemperatur gerührt. Die aminofunktionalisierten Mikrogelpartikel werden mit 200 ml Methanol gefällt, abfiltriert, mit zweimal 50 ml Methanol nachgewaschen und im Hochvakuum getrocknet. Es werden weiße Pulver erhalten.

**Tabelle 1:**

| Trimethoxyalkylamin | Abkürzung | Ausbeute [g] | Partikelgröße [nm] |
|---|---|---|---|
| N[(CH₂)₃Si(CH₃O)₃]₃ | Amin I | 5.0 | 11 |
| HN[(CH₂)₃Si(CH₃O)₃]₂ | Amin II | 4.8 | 12 |
| CH₃N[(CH₂)₃Si(CH₃O)₃]₂ | Amin III | 4.9 | 11 |
| (CH₃O)₃Si(CH₂)₃NH(CH₂)₂NH₂ | Diamin | 5.1 | 13 |

### Beispiel 2

### (Aminofunktionalisierte Organopolysiloxanpartikel)

Zu 100g der Grunddispersion I werden 6g Dimethyl-3-(methylamino)propylmethoxysilan unter Rühren zugegeben und für 15 Stunden bei Raumtemperatur gerührt. Die Dispersion wird mit 250 ml Methanol gebrochen, abfiltriert und dreimal mit jeweils 50 ml Methanol nachgewaschen. Der Filterrückstand wird in Toulol aufgenommen und mit 6 g 1,1-Dimethyl-1-Sila-2-Methyl-2-Azacyclopentan umgesetzt und bei Raumtemperatur 10 Stunden gerührt. Das Produkt wird in 200 ml Methanol ausgefällt, abfiltriert und mit zweimal 50 ml Methanol nachgewaschen und im Hochvakuum getrocknet. Es werden 8,2 g eines weißen Pulvers erhalten. Die Partikelgröße beträgt 14 nm.

### Beispiel 3

### (Phosphinofunktionalisierte Organopolysiloxanpartikel)

(alle Arbeiten finden unter Schutzgas Argon statt, die verwendeten Lösungsmittel sind entgast und anschließend mit Argon gesättigt)

Zu jeweils 100 g der entgasten und mit Argon gesättigten Grunddispersion I werden 5 g der in Tabelle 2 aufgeführten Phosphintrimethoxysilanverbindungen unter Rühren bei Raumtemperatur zudosiert. Die Dispersionen werden drei Stunden bei Raumtemperatur nachgerührt.
Die phosphinofunktionalisierten Dispersionen werden mit 6g Trimethylmethoxysilan bei Raumtemperatur versetzt. Nach weiteren 15 Stunden Rühren werden die Dispersionen mit 250 ml Methanol gebrochen und abfiltriert. Nach dreimaligem Waschen mit je 50 ml Methanol wird der Feststoff in Toluol aufgenommen, mit 8g Hexamethyldisilazan versetzt und weitere 10 Stunden bei Raumtemperatur gerührt. Die Produkte werden in 300 ml Methanol gefällt, abfiltriert, mit zweimal je 70 ml Methanol gewaschen und im Hochvakuum getrocknet. Alle Produkte sind weiße Pulver.

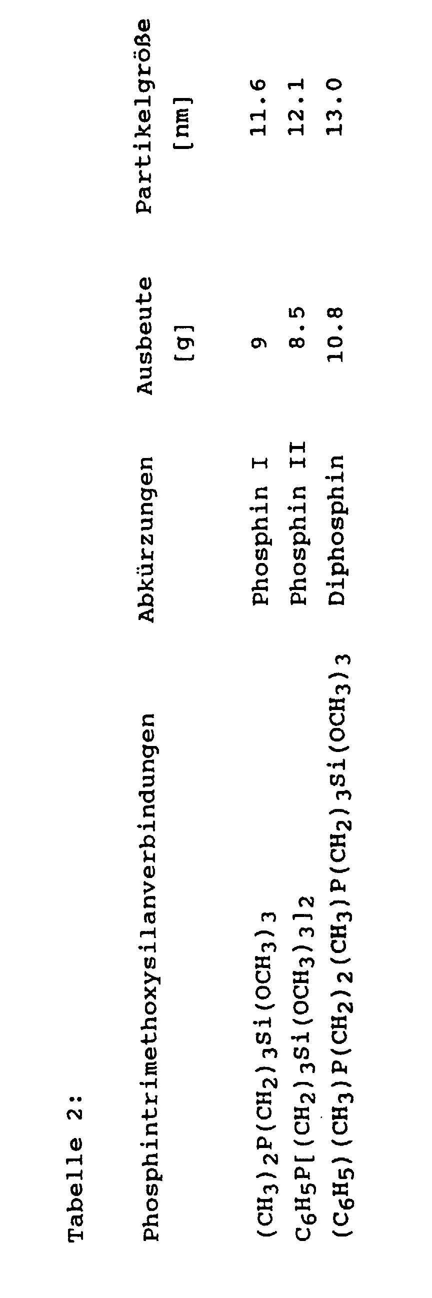

### Beispiel 4

### (Cyclodienofunktionalisierte Organopoylsiloxanpartikel)

Je 100g der Grunddispersion I werden mit je 4 g Trimethylmethoxysilan unter Rühren bei Raumtemperatur versetzt. Die Dispersionen werden für 15 Stunden gerührt, anschließend mit 200 ml Methanol gebrochen und abfiltriert. Nach dreimaligem Waschen mit je 50 ml Methanol wird der Feststoff in Toluol aufgenommen und mit 6g eines in Tabelle 3 aufgeführten Dimethyl-(3-cyclodienyl)propyl-chlorsilans versetzt und 16 Stunden bei Raumtemperatur gerührt. Zur Lösung wird danach 2g Trimethylchlorsilan zugegeben und für weitere 10 Stunden bei Raumtemperatur gerührt. Das Produkt wird mit Methanol gefällt, abfiltriert, mit zweimal 75 ml Methanol nachgewaschen und im Hochvakuum getrocknet.

**Tabelle 3:**

| Dimethyl-(3-cyclodienyl) propylchlorsilan | Abkürzung | Ausbeute [g] | Partikelgröße [nm] |
|---|---|---|---|
| (C₅H₄)(CH₂)₃Si(CH₃)₂Cl | cyclopentyl cp | 8.4 | 14.6 |
| (C₁₃H₉)(CH₂)₃Si(CH₃)₂Cl | fluorenyl flu | 7.5 | 16.8 |
| (C₉H₇)(CH₂)₃Si(CH₃)₂Cl | indenyl ind | 7.9 | 16.1 |

### Beispiel 5

### (pyridinofunktionalisierte Organopolysiloxane)

100g der Grunddispersion I werden mit 4g Trimethylmethoxysilan unter Rühren bei Raumtemperatur versetzt und für 15 Stunden gerührt. Die Dispersion wird mit 200 ml Methanol gebrochen, abfiltriert und dreimal mit 60 ml Methanol nachgewaschen. Der Filterrückstand wird in Toluol aufgenommen, mit 6g 2-Ethyl-pyridyl-dimethylchlorsilan versetzt und 16 Stunden bei Raumtemperatur gerührt. Danach wird 2 g Trimethylchlorsilan zugegeben und für weitere 10 Stunden bei Raumtemperatur gerührt. Das Produkt fällt man mit Methanol, wäscht mit zweimal 75 ml Methanol nach und trocknet im Hochvakuum. Man erhält 7.6 g eines leicht gelblichen Pulvers. Die Partikelgröße ist 14,5 nm.

### Beispiel 6

### (Titandichloridfunktionalisierte Organopolysiloxanpartikel)

100 g der Grunddispersion I werden mit 4g Trimethylmethoxysilan unter Rühren bei Raumtemperatur versetzt und 15 Stunden gerührt. Die Dispersion wird mit 200 ml Methanol gebrochen, abfiltriert und mit Methanol nachgewaschen. Der Filterrückstand wird in 500 ml Toluol gelöst. Die Lösung wird danach auf 50 ml eingeengt, wieder mit 400 ml Toluol versetzt und nochmals bis auf 50 ml eingeengt. Die verbliebene Lösung wird mit 4g Titantetrachlorid versetzt und 16 Stunden bei Raumtemperatur gerührt. Das Lösungsmittel wird abdestilliert. Der Rückstand wird im Hochvakuum getrocknet. Man erhält 9.5g Produkt.

### Beispiel 7

### (alle Arbeiten finden unter Schutzgas Argon statt, die verwendeten Lösungsmittel sind entgast und anschließend mit Argon gesättigt)

Zu 100 g der entgasten und mit Argon gesättigten Grunddispersion I werden 4g Trimethylmethoxysilan unter Rühren bei Raumtemperatur zudosiert und 15 Stunden gerührt.

Die Dispersion wird mit 200 ml Methanol gebrochen, abfiltriert und mit Methanol nachgewaschen. Der Filterrückstand wird mit den gleichen Mengen an Lösungsmitteln wie im Beispiel 6 behandelt. Die erhaltene Toluollösung wird mit Metallphosphinkomplexen der allgemeinen Formel Cl₂M[(C₆H₅)(CH₃)P(CH₂)₃Si(CH₃)₂Cl, wobei M = Pt, Pd ist, versetzt und bei Raumtemperatur 10 Stunden gerührt. Anschließend werden 2 g Trimethylchlorsilan zugegeben , weiter 10 Stunden bei Raumtemperatur gerührt und das Produkt mit 200 ml Methanol gefällt, abfiltriert, mit Methanol nachgewaschen und im Hochvakuum getrocknet.

**Tabelle 4:**

| mesoskopische Metallaminkomplexe | | | |
|---|---|---|---|
| Metall M | Abkürzung | Ausbeute [g] | Partikelgröße [nm] |
| Pt | Pt-Komplex I | 8.3 | 15.2 |
| Pd | Pd-Komplex I | 7.4 | 12.6 |

### Beispiel 8

Zu 100g der Grunddispersion I werden 5g eines Metallaminkomplexes der allgemeinen Formel Cl₂M[H₂N(CH₂)₂NH(CH₂)₃Si(OCH₃)₃], wobei M = Pt, Pd ist, unter Rühren bei Raumtemperatur zudosiert. Die Dispersionen werden für drei Stunden bei Raumtemperatur nachgerührt.

Die Dispersionen werden anschließend unter Rühren bei Raumtemperatur mit 4g Trimethylmethoxysilan versetzt. Nach weiteren 15 Stunden rühren werden die Dispersionen mit 200 ml Methanol gebrochen, abfiltriert und dreimal mit 50 ml Methanol nachgewaschen. Der Feststoff wird in Toluol aufgenommen, mit 8 g Hexamethyldisilazan versetzt und 10 Stunden bei Raumtemperatur gerührt. Die mesoskopsiche Komplexverbindung wird mit 200 ml Methanol gefällt, abfiltiert, mit zweimal 50 ml Methanol nachgewaschen und im Hochvakuum getrocknet.

**Tabelle 5:**

| Metall M | Abkürzung | Ausbeute [g] | Partikelgröße [nm] |
|---|---|---|---|
| Pt | Pt-Komplex II | 7.8 | 12.0 |
| Pd | Pd-Komplex II | 8.2 | 16.6 |

### Beispiel 9

### (Aminofunktionalisiertes Elastomerpartikel / Amin IV)

100g der Grunddispersion II werden mit 5g H₂N(CH₂)₃Si(CH₃)₂(OCH₃) unter Rühren bei Raumtemperatur versetzt und 15 Stunden weitergerührt. Die Dispersion wird in 200 ml Methanol gebrochen und abfiltriert. Der Filterrückstand wird in 70 ml Toluol aufgenommen und nochmals mit 250 ml Methanol gefällt, abfiltriert und im Hochvakuum getrocknet. Man erhält 10.2 g Produkt. Der Teilchenradius in Toluol beträgt 40 nm.

### Beispiel 10

### (Phosphinofunktionalisiertes elastomeres Organopolysiloxan)

(Alle Arbeiten finden unter Schutzgas Argon statt, die verwendeten Lösungsmittel sind entgast und anschließend mit Argon gesättigt)

100g der entgasten und mit Argon gesättigten Grunddispersion II werden mit 6g (C₆H₅)(CH₃)P(CH₂)₂Si(CH₃)₂(OCH₃) bei Raumtemperatur unter Rühren versetzt und 15 Stunden weiter gerührt. Die Dispersion wird mit 200 ml Methanol gebrochen und abfiltriert. Der Filterrückstand wird in 70 ml Toluol gelöst und nochmals mit 250ml Methanol ausgefällt, abfiltriert und im Hochvakuum getrocknet. Man erhält 8.9 g Produkt mit einem Partikelradius in Toluol von 43 nm.

### Beispiel 11

### (Phosphinofunktionalisiertes elastomeres Organopolysiloxan)

(Alle Arbeiten finden unter Schutzgas Argon statt, die verwendeten Lösungsmittel sind entgast und anschließend mit Argon gesättigt)

Zu einer Vorlage aus 125g Wasser und 1.25 g Dodecylbenzolsulfonsäure wird bei 80°C im Verlauf von 90 Minuten ein Gemisch aus 23.75g Dimethyldimethoxysilan, 0.5g Methyltrimethoxysilan und 0.6g (C₆H₅)(CH₃)P(CH₂)₂Si(OCH₃)₃ zudosiert. Die Reaktionslösung wird anschließend bei 80°C für drei Stunden gerührt.

Nach Abkühlen der Dispersion auf Raumtemperatur werden 6g Trimethylmethoxysilan zugesetzt und 15 Stunden bei Raumtemperatur gerührt. Die Dispersion wird anschließend mit 300 ml Methanol gebrochen, abfiltriert und der Filterrückstand in 100 ml Toluol aufgenommen. Das Produkt wird aus der Toluollösung mit 300 ml Methanol ausgefällt. Man erhält 16.5 g Produkt, das einen Partikelradius von 65 nm in Toluol hat.

### Beispiel 12

### (Alle Arbeiten finden unter Schutzgas Argon statt, die verwendeten Lösungsmittel sind entgast und anschließend mit Argon gesättigt)

Zu einer Vorlage aus 125g Wasser und 1.25 g Dodecybenzolsulfonsäure wird ein Gemisch von 23.75g Dimethyldimethoxysilan, 0.5g Methyltrimethoxysilan und 0.75 g eines Metallphosphinkomplexes der allgemeinen Formel Cl₂M[(C₆H₅)(CH₃)P(CH₂)₂Si(OCH₃)₃]₂, dessen Metall M in Tabelle 6 aufgeführt ist, bei Raumtemperatur innerhalb von 120 Minuten zudosiert. Die Dispersion wird für weitere drei Stunden bei Raumtemperatur gerührt.

Anschließend wird die Dispersion mit 6g Trimethylmethoxysilan unter Rühren bei Raumtemperatur versetzt und 15 Stunden nachgerührt. Die Dispersion wird in 300 ml Methanol gebrochen, abfiltriert, in 100 ml Toluol aufgenommen und nochmals mit 200 ml Methanol ausgefällt. Der Filterrückstand wird mit Methanol nachgewaschen und im Hochvakuum getrocknet.

**Tabelle 6:**

| Metallphosphinkomplexe mit mesoskopischen elastomeren Liganden | | | |
|---|---|---|---|
| Metall M | Abkürzung | Ausbeute [g] | Partikelgröße [nm] |
| Pt | Pt-Komplex III | 10.6 | 60 |
| Pd | Pd-Komplex III | 11.3 | 65 |
| Ru | Ru-Komplex I | 9.9 | 70 |
| Rh | Rh-Komplex I | 10.8 | 58 |

### Beispiel 13

### (Koordinationsverbindungen mit mesoskopischen Aminliganden)

(Alle Arbeiten finden unter Schutzgas Argon statt, die verwendeten Lösungsmittel sind entgast und anschließend mit Argon gesättigt)

Zu einer 1 %-igen Lösung eines in Tabelle 7 angegebenen mesoskopischen, aminofunktionalisierten Komplexliganden in 10 ml Toluol wird unter Rühren bei Raumtemperatur eine Metallverbindung in einem geeigneten in Tabelle 7 angegebenen Lösungsmittel zugegeben. Anschließend erhitzt man die Lösung für 4 Stunden auf 60°C. Das Produkt wird ausgefällt, abfiltriert und im Hochvakuum getrocknet. Die Ausbeuten sind in Tabelle 7 aufgeführt. cod bedeutet Cyclooctadien.

**Tabelle 7:**

| synthetisierte Komplexe mit aminfunktionellen mesoskopischen Liganden | | | | |
|---|---|---|---|---|
| eingesetzte Metallverbindung [g] | Lösungsmittel für die Metallverbindung | Komplexligand | Ausbeute [g] | Akürzung |
| [RhCl(cod)]₂ 0.25 | Ethanol/2 ml | Amin I | 0.60 | Rh-Komplex II |
| dito 0.1 | dito | Amin IV | 0.50 | Rh-Komplex III |
| PdCl₂ 0.2 | Methanol / 1ml | Amin I | 0.55 | Pd-Komplex IV |
| dito 0.1 | dito | Amin IV | 0.45 | Pd-Komplex V |
| IrCl₃ 3H₂O 0.22 | Ethanol / 2ml | Amin I | 0.5 | Ir-Komplex I |
| RuCl₃ 3H₂O 0.2 | Ethanol / 2 ml | Amin I | 0.45 | Ru-Komplex II |
| dito 0.1 | dito | Amin IV | 0.4 | Ru-Komplex III |
| H₂PtCl₆ 0.2 | Ethanol /2 ml | Amin I | 0.6 | Pt-Komplex IV |
| dito 0.1 | dito | Amin IV | 0.5 | Pt-Komplex V |
| dito 0.1 | dito | Diamin | 0.45 | Pt-Komplex VI |
| FeCl₃ 3H₂O 0.2 | Ethanol / 3ml | Amin I | 0.4 | Fe-Komplex I |
| Co(O₂CCH₃)₂ 4H₂O 0.2 | Ethanol/ 2 ml | Amin I | 0.5 g | Co-Komplex |
| NiSO₄ 6H₂O 0.2 | Ethanol/ 2ml | Diamin | 0.6 | Ni-Komplex I |
| OsCl₃ 0.2 | Ethanol / 2 ml | Amin I | 0.5 | Os-Komplex I |
| CuCl 0.1 | Suspension in Methanol / 2 ml | Amin II | 0.45 | Cu-Komplex I |
| WCl₆ 0.2 | Ethanol / 2 ml | Amin I | 0.5 | W-Komplex I |
| MoCl₅ 0.2 | Chloroform / 2ml | Amin III | 0.4 | Mo-Komplex I |
| MnSO₄ 0.2 | Methanol /2ml | Amin III | 0.55 | Mn-komplex I |
| ReCl₅ 0.2 | Ethanol/ 2ml | Amin I | 0.5 | Re-Komplex I |
| AuCl₃ 0.2 | Ethanol / 2ml | Amin I | 0.4 | Au-Komplex I |
| AgNO₃ 0.2 | Methanol / 2ml | Amin I | 0.4 | Ag-Komplex I |

### Beispiel 14

### (gemischte Koordinationsverbindungen mit mesoskopischen Aminoliganden)

(Alle Arbeiten finden unter Schutzgas Argon statt, die verwendeten Lösungsmittel sind entgast und anschließend mit Argon gesättigt)

Zu einer Vorlage aus 20 ml Toluol und 0.5g des mesoskopischen Aminoligandenen (Amin I) aus dem Beispiel 1 wird unter Rühren bei Raumtemperatur eine Mischung aus 0.lg H₂PtCl₆ und 0.lg RuCl₃ in 3ml Methanol gegeben. Anschließend wird weiter 4 Stunden bei 60°C gerührt. Das Produkt wird mit Methanol ausgefällt, abfiltriert und im Hochvakuum getrocknet. Man erhält 0.55 g Produkt.

### Beispiel 15

### (mesoskopische Koordinationsverbindungen mit Phosphinoliganden)

(Alle Arbeiten finden unter Schutzgas Argon statt, die verwendeten Lösungsmittel sind entgast und anschließend mit Argon gesättigt)

Zu einer Vorlage aus 20 ml Toluol und 0.5g eines mesoskopischen Komplexliganden aus Beispiel 3 wird unter Rühren bei Raumtemperatur eine in Tabelle 8 aufgeführte Metallverbindung in einem geeigneten Lösungsmittel zugegeben. Die Reaktionslösung wird für weitere vier Stunden gerührt. Das Produkt wird mit Methanol ausgefällt, abfiltriert und im Hochvakuum getrocknet.

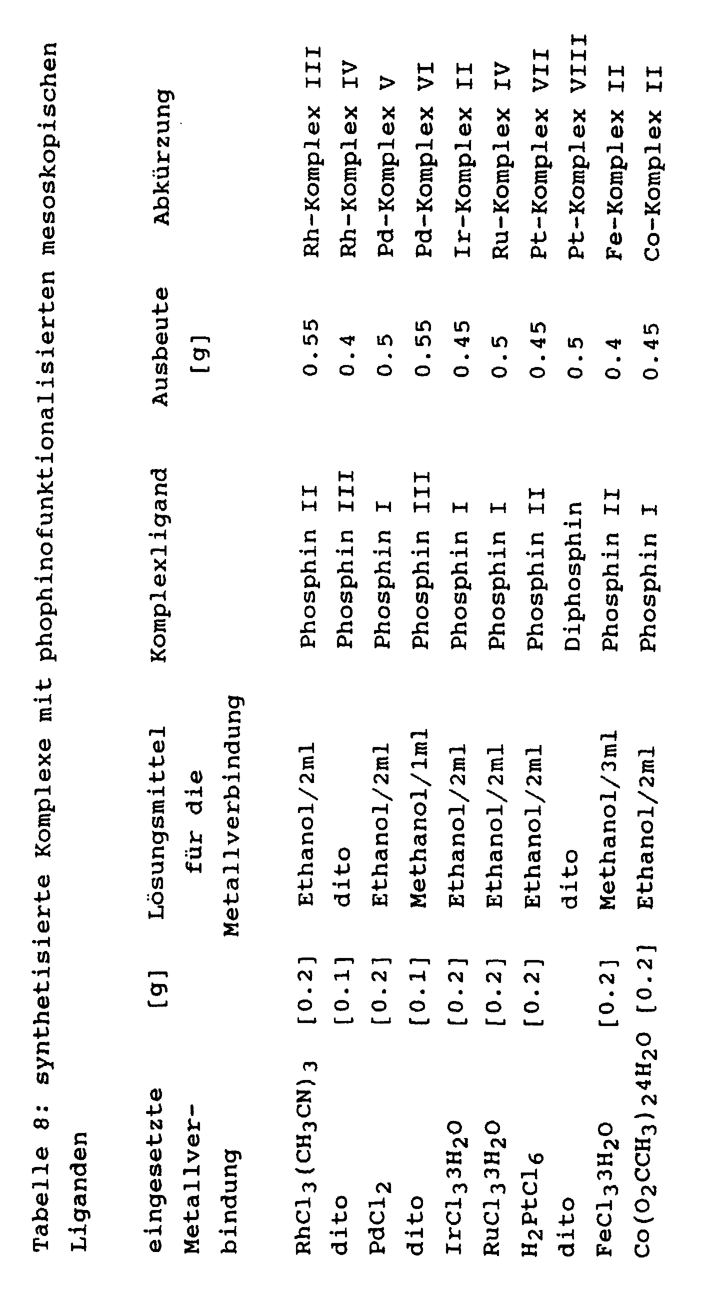

### Beispiel 16

### (Koordinationsverbindungen mit mesoskopischen Cyclodienliganden)

(Alle Arbeiten finden unter Schutzgas Argon statt, die verwendeten Lösungsmittel sind entgast und anschließend mit Argon gesättigt)

1g der in Beispiel 4 hergestellten cyclodienfunktionalisierten Organopolysiloxanpartikel wird in 50 ml Diethylether bei Raumtemperatur gelöst und mit 2.0 ml Butyllithium (1.6 M in Hexan) versetzt. Nach 10 Minuten Rühren gibt man 0.4 g Cp₂MCl₂, wobei M = Zr; Hf; Ti, zu und rührt weitere 30 Minuten. Die erhaltenen Suspension wird über Natriumsulfat filtriert und mit zweimal 30 ml Ether nachgewaschen. Die Metallocenkomplexverbindung wird mit Methylenchlorid vom Natriumsulfat abgelöst und das Lösungsmittel wird anschließend wieder im Hochvakuum abgezogen.

**Tabelle 9:**

| Metallocenkomplexverbindungen | | | |
|---|---|---|---|
| Zentralatom M | mesoskopischer Ligand | Ausbeute [g] | Abkürzung |
| Ti | Ind | 1.1 | Ti-Komplex I |
| | Flu | 1.0 | Ti-Komplex II |
| Zr | Ind | 1.2 | Zr-Komplex I |
| | Flu | 1.1 | Zr-Komplex II |
| Hf | Flu | 1.0 | Hf-Komplex I |

### Beispiel 17

### (Koordinationsverbindungen mit mesoskopischen heterocyclischen Liganden)

1g der in Beispiel 5 hergestellten pyridinofunktionalisierten Organopolysiloxanpartikel werden in Toluol gelöst und mit in Tabelle 10 aufgeführten Metallverbindungen, die in geeigneten Lösungsmitteln gelöst oder suspendiert sind, versetzt. Das Reaktionsgemisch wird 4 Stunden bei 70°C gerührt. Die erhaltenen Komplexverbindungen werden mit Methanol ausgefällt, abfiltriert und im Hochvakuum getrocknet.

**Tabelle 10:**

| synthetisierte Komplexverbindungen mit mesoskopischen Pyridinoliganden | | | | |
|---|---|---|---|---|
| Metallverbindung | [g] | Lösungsmittel für die Metallverbindung | Ausbeute [g] | Abkürzung |
| H₂PtCl₆ | [0.2] | Methanol/2ml | 1.1 | Pt-Komplex IX |
| CuCl | | suspendiert in Methanol/3ml | 1.0 | Cu-Komplex II |

### Beispiel 18

### (Hydroformylierung)

0.4 g der Rh-Komplexes III aus Beispiel 15 werden in 500 ml 1-Octen gelöst und in einen Autoklaven gegeben. Die Reaktion wird bei einem totalen Druck von 200 bar, einer Temperatur von 100°C und einem H₂ : CO Verhältnis von 1:1 geführt.

Die gaschromatographische Analyse (GC) ergibt 98 % Umsatz des Octens zum entsprechenden Aldehyd.

### Beispiel 19

### (Hydrierung von Mehrfachbindungen)

0.4 g des Pd-Komplexes V werden in 100 ml 1-Octen gelöst und in einem 1 1 Autoklaven gegeben. Bei einem konstanten H₂ Druck von 5bar wird das Reaktionsgemisch auf 60°C erwärmt und für 5 Stunden gerührt.

Die GC-Analyse ergibt, daß 95 % des 1-Octens zu Octan hydriert werden.

### Beispiel 20

### (Hydrosilylierung)

0.5 g der Pt-Komplexes II werden in 22.15 g 1-Octen und 26.73 g HSiCl₃ gelöst. Die Reaktionslösung wird in einen Glasautoklaven gegeben und für 24 Stunden unter Rühren auf 100°C erhitzt.

Die GC-Analyse des Proukts ergibt, daß 95% des eingesetzten 1-Octens zu Octyltrichlorsilan abreagiert.

### Beispiel 21

### (Olefinpolymerisation)

(Alle Arbeiten finden unter Schutzgas Argon statt, die verwendeten Lösungsmittel sind entgast und anschließend mit Argon gesättigt)

### Aktivierung der Katalysatorvorstufe

50 mg der jeweiligen mesoskopischen Metallocendichloridkomplexe(Ti-Komplex II, Zr-Komplex I) aus Beispiel 16 werden in 10 ml Toluol gelöst und mit 2 ml Methylaluminiumoxid, 30 Gew.-% in Toluol, M_{w} = 1100 g/mol, versetzt.

### Olefinpolymerisierung

In einem 1 l Autoklaven werden 500 ml Pentan bei 10°C vorgelegt, mit 10 ml der Katalysatorlösung in Toluol versetzt und ein Ethylendruck von 10 bar angelegt. Das Reaktionsgemsich wird unter Rühren eine Stunde bei 60°C gehalten. Das erhaltenen Polymer wird abfiltriert, mit verdünnter Natronlauge, Wasser und Aceton gewaschen und im Trockenschrank werden Lösungsmittelreste entfernt.

**Tabelle 11:**

| Ausbeuten der Olefinpolymerisation mit mesoskopischen Metallocenkatalysatoren | |
|---|---|
| mesoskopischer Metallocendichloridkomplex | Ausbeute an Polyethylen [g] |
| Ti-Komplex II | 30.5 |
| Zr-Komplex I | 35.5 |

### Beispiel 22

### (Durch Strahlungsinduktion katalytisch aktivierte mesoskopische Katalysatorsysteme)

0.4g des Platinkomplexes VI werden in 5 ml Methanol suspendiert und mit 5 ml einer 0.1%-igen Lösung von 1-Phenyl-3-cyclohexyltriazenoxidkaliumsalz in Methanol versetzt und bei Raumtemperatur zwei Stunden gerührt. Der Feststoff wird abfiltriert und mehrmals mit Toluol extrahiert. Die Toluollösung wird am Rotationsverdampfer eingeengt und das erhaltene Produkt bei Raumtemperatur im Hochvakuum getrocknet. Man erhält 0.3 g des Platinkomplexes X.

0.05 g des Platinkomplexes X werden in 5.4 g 1-Octen gelöst und mit 8.2 g Triethoxysilan bei Raumtemperatur unter Rühren versetzt. Die Hydrosilylierungsreaktion wird durch UV-Bestrahlung initiiert (15 Sekunden mit einer Wellenlänge von 350nm). Die Reaktionslösung wird für weitere 30 Minuten nachgerührt.

Die GC-Analyse des Produktes ergibt, daß 96.5% des eingesetzten 1-Octens zu Octyltriethoxysilan abreagiert.

## Patentansprüche

1. Aus einem einzigen Molekül bestehende vernetzte Organopolysiloxanpartikel, die chemisch angebundene Metallverbindungen aufweisen, einen über statische und dynamische Lichtstreuung ermittelbaren mittleren Durchmesser von 5 bis 200 nm besitzen und die in mindestens einem der Lösungsmittel, die ausgewählt werden aus Dichlormethan, Pentan, Aceton, Ethanol und Wasser zu mindestens 1 Gew.% löslich sind, wobei mindestens 80 % der Partikel einen Durchmesser besitzen, der höchstens 30 % vom mittleren Durchmesser abweicht.

2. Organopolysiloxanpartikel nach Anspruch 1, bei denen die mittleren Molmassen M_{w} 10⁵ bis 10¹⁰ g/mol betragen.

3. Organopolysiloxanpartikel nach Anspruch 1 oder 2, bei denen die Metallverbindungen, an die Organopolysiloxanpartikel chemisch angebunden sind, ausgewählt werden aus den Metallen der I., IV., VI., VII und VIII. Nebengruppe.

4. Organopolysiloxanpartikel nach einem der Ansprüche 1 bis 3, die an der Oberfläche Einheiten aufweisen, die ausgewählt werden aus Einheiten der allgemeinen Formeln
[AR₂SiO_{1/2}] **(1),**
[ARSiO_{2/2}] **(2)**
und
[ASiO_{3/2}] **(3),**
und die restlichen Einheiten der Organopolysiloxanpartikel bestehen aus 0,5 bis 80,0 Gew.-% Einheiten der allgemeinen Formel
[R₃SiO_{1/2}] **(4),**
0 bis 99,0 Gew.-% Einheiten der allgemeinen Formel
[R₂SiO_{2/2}] **(5),**
0 bis 99,5 Gew.-% Einheiten der allgemeinen Formel
[RSiO_{3/2}] **(6)**
und 0 bis 80,0 Gew.-% Einheiten der allgemeinen Formel
[SiO_{4/2}] **(7),**
wobei
**A** eine Ligandeneinheit der allgemeinen Formeln
spL' **(8)**
oder
spsp'L" **(9)**
ist, wobei mindestens eine Einheit der allgemeinen Formeln (8) oder (9) pro Organopolysiloxanpartikel in einem Komplex der allgemeinen Formeln
spL"ᵢMLₖ **(10)**
oder
spsp'L"ᵢMLₖ **(11)**
gebunden ist, und wobei
**M** ein Metall der I., IV., VI., VII. und VIII. Nebengruppe des periodischen Systems der Elemente,
**L** einen Komplexliganden in der Koordinationssphäre des Metalls **M**,
**L'** einen über einen Spacer **sp** an die Oberfläche eines Organopolysiloxanpartikels gebundenen Komplexliganden in der Koordinationssphäre des Metalls **M**,
**L"** einen über zwei Spacer **sp** und **sp'** an die Oberfläche eines Organopolysiloxanpartikels gebundenen Komplexliganden in der Koordinationssphäre des Metalls **M**,
**sp** und **sp'** gleiche oder verschiedene zweiwertige SiC-gebundene, gegebenenfalls substituierte C₀- bis C₁₈-Kohlenwasserstoffreste, welche durch zweibindige, beidseitig an Kohlenstoffatome gebundene Reste aus der Gruppe -O-, -COO-, -OOC-, -CONR²-, -NR²CO-, -CO- und-[SiR₂]ₗ- unterbrochen sein können,
**R** gleiche oder verschiedene einwertige SiC-gebundene, gegebenenfalls halogensubstituierte C₁- bis C₁₈-Alkylreste,
**i** Werte von 1 bis höchstens die Koordinationszahl des Metalls **M**,
**k** Werte von 0 bis höchstens die Koordinationszahl des Metalls **M** minus i und
**l** Werte von 1 bis 100 bedeuten.

5. Verfahren zur Herstellung von vernetzten Organopolysiloxanpartikeln, die an der Oberfläche chemisch gebundene Metallatome aufweisen, gemäß einem der Ansprüche 1 bis 4, bei dem in einem ersten Schritt durch Zudosieren von Silanen der allgemeinen Formel (12)
RₐSi(OR²)₄₋ₐ **(12),**
und gegebenenfalls siliciumorganischen Verbindungen, die aufgebaut sind aus Einheiten der allgemeinen Formel (13)
R_{b}(R²O)_{c}SiO_{(4-b-c)/2} **(13),**
zu einer bewegten Mischung aus Emulgator und Wasser eine kolloidale Suspension von Organopolysiloxanpartikeln hergestellt wird,
in einem zweiten Schritt die kolloidale Suspension mit Silanen der allgemeinen Formel (14)
AR_{d}Si(OR²)_{4-d} **(14),**
und/oder siliciumorganischen Verbindungen, die aufgebaut sind aus Einheiten der allgemeinen Formel (15)
AₑR_{f}(R²O)_{g}SiO_{(4-e-f-g)/2} **(15),**
versetzt wird,
mit der Maßgabe, daß die Verbindungen, die aufgebaut sind aus Einheiten der allgemeinen Formel (15),
mindestens einen Rest A und einen Rest (R²O) aufweisen, und in einem dritten Schritt die kolloidale Suspension mit siliciumorganischer Verbindung der allgemeinen Formel (16)
(R³R⁴ ₂Si)ₕX **(16),**
versetzt wird, mit der Maßgabe, daß die siliciumorganischen Verbindungen der allgemeinen Formel (16) wasserlöslich sind oder in Wasser zu einer wasserlöslichen Verbindung hydrolysieren,
wobei
**R**^{**2**} und **R**^{**4**} die Bedeutungen von **R**,
**R**^{**3**} die Bedeutungen von **A** oder **R**,
**X** für die Bedeutungen **h** = 1, -OR⁵,
-ONR⁵₂ oder -OOCR⁵ und
für die Bedeutungen **h** = 2 -O- oder -S-,
**R**^{**5**} die Bedeutungen von **R**,
**a** die Werte 0, 1, 2 oder 3,
**b** und **c** jeweils unabhängig voneinander die Werte 0, 1, 2, 3 oder 4,
**d** die Werte 0, 1, oder 2,
**e, f** und **g** jeweils unabhängig voneinander die Werte 0, 1, 2 oder 3,
**h** die Werte 1 oder 2 bedeuten und
**R** und **A** die in Anspruch 4 angegebenen Bedeutungen
aufweisen.

6. Verfahren nach Anspruch 5, bei dem Organopolysiloxanpartikel, die in der Summe mehr als 15 Gew.-% an Einheiten der allgemeinen Formeln (6) und (7) enthalten, nach Isolierung im Anschluß an den dritten Schritt in einem vierten Reaktionsschritt in einem aprotischen Lösungsmittel mit siliciumorganischer Verbindung der allgemeinen Formeln (17)
(R⁶R⁷ ₂Si)ᵢY **(17),**
oder (18) 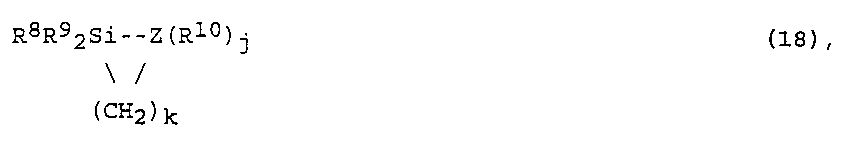 versetzt werden, wobei
**R**^{**6**} und **R**^{**8**} die Bedeutungen von **A** oder **R**,
**R**^{**7**}**, R**^{**9**} und **R**^{**10**} die Bedeutungen von **R**,
**Y** für die Bedeutung **i** = 1, Halogenatome,
-OR⁶, -NR⁶₂, -ONR⁶₂ oder -OOCR⁶ und
für die Bedeutung **i** = 2 -O-, =N(R⁶) oder -S-,
**Z** für die Bedeutung **j** = 1 -N- und
für die Bedeutung **j** = 0 -O- oder -S-,
**i** die Werte 1 oder 2,
**j** die Werte 0 oder 1,
**k** die Werte 1 bis 30 bedeuten und
**R** und **A** die in Anspruch 4 angegebenen Bedeutungen
aufweisen.

7. Verfahren zur Herstellung von elastomeren vernetzten Organopolysiloxanpartikeln, die chemisch gebundene Metallverbindungen aufweisen, gemäß einem der Ansprüche 1 bis 4, bei dem aus zusammen mindestens 80 Mol-% Silanen der allgemeinen Formeln (12), in der a den Wert 2 hat und (14), in der **d** den Wert 1 hat, der erste und zweite Schritt gemäß Anspruch 5 zu einem Schritt zusammengefaßt werden.

8. Verwendung der Organopolysiloxanpartikel gemäß einem der Ansprüche 1 bis 4 als Katalysatoren.

## Claims

1. Crosslinked organopolysiloxane particles which comprise a single molecule, carry chemically bonded metal compounds, have an average diameter of 5 to 200 nm which can be determined via static and dynamic light scattering and are soluble to the extent of at least 1% by weight in at least one of the solvents chosen from the group consisting of methylene chloride, pentane, acetone, ethanol and water, at least 80% of the particles having a diameter which deviates not more than 30% from the average diameter.

2. Organopolysiloxane particles according to Claim 1, in which the average molecular weights M_{w} are 10⁵ to 10¹⁰ g/mol.

3. Organopolysiloxane particles according to Claim 1 or 2, in which the metal compounds to which the organopolysiloxane particles are bonded chemically are chosen from the metals of subgroup I, IV, VI, VII and VIII.

4. Organopolysiloxane particles according to one of Claims 1 to 3, which have, on the surface, units which are chosen from units of the general formulae
[AR₂SiO_{1/2}] (1),
[ARSiO_{2/2}] (2)
and
[ASiO_{3/2}] (3),
and the remaining units of the organopolysiloxane particles comprise
0.5 to 80.0% by weight of units of the general formula
[R₃SiO_{1/2}] (4),
0 to 99.0% by weight of units of the general formula
[R₂SiO_{2/2}] (5),
0 to 99.5% by weight of units of the general formula
[RSiO_{3/2}] (6)
and
0 to 80.0% by weight of units of the general formula
[SiO_{4/2}] (7),
in which
A is a ligand unit of the general formula
spL' (8),
or
spsp'L" (9)
at least one unit of the general formulae (8) or (9) per organopolysiloxane particle being bonded in a complex of the general formulae
spL"ᵢMLₖ (10)
or
spsp'L"ᵢMLₖ (11)
and in which
M is a metal of subgroup I, IV, VI, VII or VIII of the Periodic Table of the Elements,
L is a complexing ligand in the coordination sphere of the metal M,
L' is a complexing ligand in the coordination sphere of the metal M bonded via a spacer sp to the surface of an organopolysiloxane particle,
L" is a complexing ligand in the coordination sphere of the metal M bonded via two spacers sp and sp' to the surface of an organopolysiloxane particle,
sp and sp' are identical or different bivalent SiC-bonded, optionally substituted C₀- to C₁₈-hydrocarbon radicals, which can be interrupted by divalent radicals, bonded to carbon atoms on both sides, from the group consisting of -O-, -COO-, -OOC-, -CONR²-, -NR²CO-, -CO- and -[SiR₂]₁-,
R is identical or different monovalent SiC-bonded, optionally halogen-substituted C₁- to C₁₈-alkyl radicals,
i has values from 1 to not more than the coordination number of the metal M,
k has values from 0 to not more than the coordination number of the metal M minus i and
l has values from 1 to 100.

5. Process for the preparation of crosslinked organopolysiloxane particles which carry metal atoms bonded chemically to the surface, according to one of Claims 1 to 4, in which, in a first step, by metering silones of the general formula (12)
RₐSi(OR²)₄₋ₐ (12),
and, if appropriate, organosilicon compounds which are built up from units of the general formula (13)
R_{b}(R²O)_{c}SiO_{(4-b-c)/2} (13),
into an agitated mixture of emulsifier and water, a colloidal suspension of organopolysiloxane particles is prepared,
in a second step, silanes of the general formula (14)
AR_{d}Si(OR²)_{4-d} (14),
and/or organosilicon compounds which are built up from units of the general formula (15)
AₑR_{f} (R²O)_{g}SiO_{(4-e-f-g)/2} (15),
are added to the colloidal suspension,
with the proviso that the compounds which are built up from units of the general formula (15) contain at least one radical A and one radical (R²O),
and, in a third step, an organosilicon compound of the general formula (16)
(R³R⁴ ₂Si)ₕX (16),
is added to the colloidal suspension, with the proviso that the organosilicon compounds of the general formula (16) are water-soluble or hydrolyse in water to give a water-soluble compound,
in which
R² and R⁴ have the meanings of R,
R³ has the meanings of A or R,
X, if h = 1, is -OR⁵, -ONR⁵ ₂ or -OOCR⁵ and if h = 2, is -O- or -S-,
R⁵ has the meanings of R,
a has the values 0, 1, 2 or 3,
b and c, in each case independently of one another, have the values 0, 1, 2, 3 or 4,
d has the values 0, 1 or 2,
e, f and g, in each case independently of one another, have the values 0, 1, 2 or 3,
h has the values 1 or 2 and
R and A have the meanings given in Claim 4.

6. Process according to Claim 5, in which, after isolation after the third step, organopolysiloxane particles which comprise more than 15% by weight in total of units of the general formulae (6) and (7) are treated, in a fourth reaction step in an aprotic solvent, with an organosilicon compound of the general formulae (17)
(R⁶R⁷ ₂Si)ᵢY (17),
or (18)
R⁸R⁹ ₂Si--Z(R¹⁰)ⱼ (18),
(CH₂)ₖ
in which
R⁶ and R⁸ have the meanings of A or R,
R⁷, R⁹ and R¹⁰ have the meanings of R,
Y, if i = 1, is halogen atoms, -OR⁶, -NR⁶₂, -ONR⁶₂ or -OOCR⁶ and
if i = 2, is -O-, =N(R⁶) or -S-,
Z, if j = 1, is -N- and
if j = 0, is -O- or -S-,
i has the values 1 or 2,
j has the values 0 or 1,
k has the values from 1 to 30 and
R and A have the meanings given in Claim 4.

7. Process for the preparation of elastomeric crosslinked organopolysiloxane particles which carry chemically bonded metal compounds, according to one of Claims 1 to 4, in which the first and second step according to Claim 5 are combined from at least 80 mol% together of silanes of the general formulae (12), in which a has the value 2, and (14) in which d has the value 1, to give one step.

8. Use of organopolysiloxane particles according to one of Claims 1 to 4 as catalysts.

## Revendications

1. Particules d'organopolysiloxane réticulées composées d'une seule molécule, qui présentent des composés métalliques chimiquement liés, possèdent un diamètre moyen de 5 à 200 nm qui peut être déterminé par dispersion statique et dynamique de lumière, et sont solubles à au moins 1% en poids dans au moins un des solvants qui sont choisis parmi le dichlorométhane, le pentane, l'acétone, l'éthanol et l'eau, au moins 80% des particules possédant un diamètre qui s'écarte de 30% au maximum du diamètre moyen.

2. Particules d'organopolysiloxane suivant la revendication 1, dans lesquelles les masses molaires moyennes M_{w} valent 10⁵ à 10¹⁰ g/mole.

3. Particules d'organopolysiloxane suivant la revendication 1 ou 2, dans lesquelles les composés métalliques, auxquels les particules d'organopolysiloxane sont chimiquement liées, sont choisis parmi les métaux des sous-groupes I, IV, VI, VII et VIII.

4. Particules d'organopolysiloxane suivant l'une des revendications 1 à 3, qui présentent en surface des unités qui sont choisies parmi des unités de formules générales
[AR₂SiO_{1/2}] (1),
[ARSiO_{2/2}] (2),
et
[ASiO_{3/2}] (3),
et les unités restantes des particules d'organopolysiloxane sont composées de 0,5 à 80,0% en poids d'unités de formule générale
[R₃SiO_{1/2}] (4),
de 0 à 99,0% en poids d'unités de formule générale
[R₂SiO_{2/2}] (5),
de 0 à 99,5% en poids d'unités de formule générale
[RSiO_{3/2}] (6)
et de 0 à 80,0% en poids d'unités de formule générale
[SiO_{4/2}] (7),
dans lesquelles
A est une unité de ligand de formules générales
spL' (8)
ou
spsp'L" (9),
dans laquelle au moins une unité de formules générales (8) ou (9) par particule d'organopolysiloxane est liée dans un complexe de formules générales
spL"ᵢMLₖ (10)
ou
spsp'L"ᵢMLₖ (11),
et dans lesquelles
M signifie un métal des sous-groupes I, IV, VI, VII et VIII du système périodique des éléments;
L signifie un ligand complexant dans la sphère de coordination du métal M;
L' signifie un ligand complexant, dans la sphère de coordination du métal M, lié à la surface d'une particule d'organopolysiloxane par l'intermédiaire d'un radical intercalaire sp;
L" signifie un ligand complexant, dans la sphère de coordination du métal M, lié à la surface d'une particule d'organopolysiloxane par l'intermédiaire de deux radicaux intercalaires sp et sp';
sp et sp' signifient des radicaux hydrocarbonés en C₀ à C₁₈ divalents identiques ou différents, à liaison SiC, le cas échéant substitués, qui peuvent être interrompus par des radicaux divalents, liés des deux côtés à des atomes de carbone, du groupe -O-, -COO-, -OOC-, -CONR²-, -NR²CO-, -CO- et -[SiR₂]₁-;
R signifie des radicaux alkyle en C₁ à C₁₈ monovalents identiques ou différents, à liaison SiC, le cas échéant substitués par halogène;
i vaut de 1 à au plus l'indice de coordination du métal M;
k vaut de 0 à au plus l'indice de coordination du métal M moins i, et
l vaut de 1 à 100.

5. Procédé de préparation de particules d'organopolysiloxane réticulées qui présentent en surface des atomes métalliques chimiquement liés, suivant l'une des revendications 1 à 4, dans lequel, dans une première étape, on prépare une suspension colloïdale de particules d'organopolysiloxane par l'addition de silanes de formule générale (12)
RₐSi(OR²)₄₋ₐ (12),
et le cas échéant de composés organosiliciés, qui sont constitués d'unités de formule générale (13)
R_{b}(R²O)_{c}SiO_{(4-b-c)/2} (13),
à un mélange sous agitation d'émulsionnant et d'eau; dans une deuxième étape, on ajoute à la suspension colloïdale des silanes de formule générale (14)
AR_{d}Si(OR²)_{4-d} (14),
et/ou des composés organosiliciés qui sont constitués d'unités de formule générale (15)
AₑR_{f}(R²O)_{g}SiO_{(4-e-f-g)/2} (15),
étant entendu que les composés qui sont constitués d'unités de formule générale (15) présentent au moins un radical A et un radical (R²O), et
dans une troisième étape, on ajoute à la suspension colloïdale un composé organosilicié de formule générale (16)
(R³R⁴ ₂Si)ₕX (16),
étant entendu que les composés organosiliciés de formule générale (16) sont solubles dans l'eau ou s'hydrolysent dans l'eau en un composé soluble dans l'eau,
dans lesquelles
R² et R⁴ ont les significations de R;
R³ a les significations de A ou de R;
X pour h = 1, a les significations -OR⁵, -ONR⁵₂ ou -OOCR⁵ et, pour h = 2, a les significations -O- ou -S-;
R⁵ a les significations de R;
a vaut 0, 1, 2 ou 3;
b et c ont dans chaque cas indépendamment l'un de l'autre les valeurs 0, 1, 2, 3 ou 4;
d a les valeurs 0, 1 ou 2;
e, f et g ont dans chaque cas indépendamment l'un de l'autre les valeurs 0, 1, 2 ou 3;
h a les valeurs 1 ou 2, et
R et A ont les significations données à la revendication 4.

6. Procédé suivant la revendication 5, dans lequel on ajoute aux particules d'organopolysiloxane, qui contiennent au total plus de 15% en poids d'unités de formules générales (6) et (7), après isolement à la suite de la troisième étape, dans une quatrième étape de réaction, dans un solvant aprotique, un composé organosilicié de formules générales (17)
(R⁶R⁷ ₂Si)ᵢY (17),
ou (18) 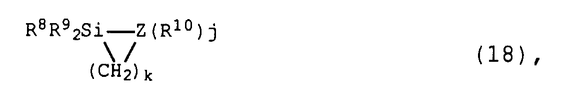 dans lesquelles
R⁶ et R⁸ ont les significations de A ou R;
R⁷, R⁹ et R¹⁰ ont les significations de R;
Y pour i = 1, signifie des atomes d'halogène, -OR⁶, -NR⁶₂, -ONR⁶₂ ou -OOCR⁶ et,
pour i = 2, signifie -O-, =N(R⁶) ou -S-;
Z pour j = 1, signifie -N- et,
pour j = 0, signifie -O- ou -S-;
i a les valeurs 1 ou 2;
j a les valeurs 0 ou 1;
k a les valeurs 1 à 30, et
R et A ont les significations données à la revendication 4.

7. Procédé de préparation de particules d'organopolysiloxane réticulées élastomères, qui présentent des composés métalliques chimiquement liés, suivant l'une des revendications 1 à 4, dans lequel la première et la deuxième étapes suivant la revendication 5 sont combinées en une étape à partir d'au moins 80% en mole au total de silanes de formules générales (12), dans laquelle a a les valeurs 2, et (14), dans laquelle d a la valeur 1.

8. Utilisation des particules d'organopolysiloxane suivant l'une des revendications 1 à 4 comme catalyseurs.
